# EUROPEAN PATENT APPLICATION

(11) **EP 2 359 876 A1**
(43) Date of publication of application: **24.08.2011**
(21) Application number: 10001486.9
(22) Date of filing: 12.02.2010
(51) Int. Cl.: A61L 27/16, A61L 27/50, A61L 31/04

(54) **Medical device comprising a porous article of ePTFE exhibiting improved cellular tissue ingrowth**

(71) Applicant: Aesculap AG, 78532 Tuttlingen/Donau (DE)
(72) Inventor: Goldmann, Helmut, 78532 Tuttlingen (DE); Langanke, Dennis, 78532 Tuttlingen (DE)
(74) Representative: Patentanwälte Ruff, Wilhelm, Beier, Dauster & Partner

(57) **Abstract**

The present invention relates to a medical device comprising a porous article of expanded polytetrafluoroethylene (ePTFE) having a microstructure comprising nodes interconnected by fibrils, wherein the microstructure at a surface of the porous article at least partially comprises nodes having segments that are free of fibrillate interconnections. Further, the invention relates to a method for the manufacture of the medical device.

## Description

The present invention relates generally to a medical device comprising a porous article of expanded polytetrafluoroethylene (ePTFE) having a microstructure at the surface of the article promoting tissue ingrowth and a method for manufacturing the medical device.

Generally, vascular prostheses should have an inside surface that is as smooth as possible in order to avoid platelet activation and adhesion that might lead to deposit of fibrin layers on the inside surface finally causing occlusion of the prostheses. Besides, vascular prostheses should have an outside surface that facilitates tissue ingrowth in order to provide a secure anchoring of the prosthesis with surrounding tissues of a human or animal body.

It is already known that fibroblasts readily enter a vascular prosthesis made of knitted or woven fabric based on polyester that is due to the loose structure of the tubular wall of such a prosthesis. However, knitted or woven prostheses generally suffer from the drawback that bleeding occurs through the wall immediately after implantation which results in undesired platelet activation and adhesion finally leading to occlusion of the prostheses. Besides, blood trapped by the prosthesis structure may cause the manifestation of seroma that is normally associated by an inflammatory response. Thus, macrophages may be attracted that may lead to a degradation of the prosthesis.

Further, it is known to use vascular prostheses made of expanded polytetrafluoroethylene (ePTFE) having a porous microstructure which is defined by nodes interconnected by fibrils. Known ePTFE prostheses normally have a pore structure at the inside surface that satisfactorily lowers the risk of platelet activation and subsequent adhesion on the inside surface. However, due to the above mentioned node and fibril microstructure of ePTFE the entry of fibroblasts into the prostheses is generally deficient and normally lasts over a long period of time. Accordingly, several attempts were made to optimize the ingrowth of fibroblast into ePTFE prostheses. For instance, reference is made to the documents US 4,208,745, US 4,877,661, US 4,713,070 and the US 5,433,909. However, often laborious and time-consuming techniques are necessary without significantly improving the cellular entry into ePTFE prostheses.

Accordingly, it is an object of the present invention to provide a medical device made of ePTFE having an optimized microstructure at its surface facilitating an easy natural tissue ingrowth from the outer periphery of the medical device that is preferably comparable to the tissue ingrowth characteristics of known knitted or woven vascular grafts without increasing the risk for the patient.

This problem is solved by a medical device having the features of independent claim 1. Preferred embodiments of the device are the object of dependent claims 2 to 12. The present invention further encompasses a method for the manufacture of the medical device according to independent claim 13. A preferred embodiment of the method is the object of dependent claim 14. A medical device that is obtained or obtainable by the afore-mentioned method is the object of independent claim 15. The wording of all of the claims is incorporated in the present description by reference.

The medical device according to the present invention comprises a porous article of expanded polytetrafluoroethylene (ePTFE) having a microstructure comprising interspaced nodes interconnected by fibrils, wherein the microstructure at a surface of the article, preferably at an outside surface thereof, at least partially comprises nodes having segments (node segments) that are free of fibrillate interconnections (crosslinks). Preferably, the nodes having segments being free of fibrillate interconnections are only present at the surface, in particular an outside surface, of the porous article.

Conventional ePTFE prostheses are featured by a microfibrous structure composed of interspaced nodes interconnected by fibrils. The medical device according to the present invention differs from the conventional prostheses in that its microstructure at the surface of the article, preferably at an outside surface thereof, includes node segments that are not interconnected by fibrils (i.e. are free of fibrillate interconnections). In other words, the surface of the porous article, preferably an outside surface thereof, is free of fibrillate interconnections. Thus, the corresponding internodal spaces are restricted to a less extent by fibrillate interconnections. This leads to the manifestation of enlarged cavities or openings, in particular pores, at the surface of the article, facilitating an easier tissue entry into the article from its outer periphery. This is in favour for a secure anchoring of the device. Furthermore, the healing process after implantation of the medical device is supported by an easier tissue entry into the device.

The term "polytetrafluoroethylene" as used herein also comprises a copolymer of tetrafluoroethylene.

Accordingly, the term "copolymer" as used herein preferably means a polymer that comprises at least two different monomer units.

The term "copolymer of tetrafluoroethylene" as used herein encompasses a polymer that is composed of at least one further monomer unit along tetrafluoroethylene. Such a further monomer unit may be selected from the group consisting of chlorotrifluoroethylene, perfluoroalkoxytetrafluoroethylene, hexafluoropropylene, tetrafluoropropylene and combinations thereof.

Preferably, the node segments being free of fibrillate interconnections are aligned (oriented) to the surface of the article. In particular, the node segments that are free of fibrillate interconnections form an outer periphery of the porous article.

In a preferred embodiment, the node segments that are free of fibrillate interconnections protrude, preferably pillar-like, from the surface of the porous article. Accordingly, these node segments, effect a roughly structured surface that promotes tissue entry, typically connective tissue entry, from the outer periphery of the article. Particularly, the node segments being free of fibrillate interconnections facilitating the trapping of body cells, in particular of fibroblasts, triggering cellular entry into the article. More preferably, the node segments that are free of fibrillate interconnections confer the surface of the article a velour-like appearance.

According to a further embodiment, the nodes at the surface of the porous article are predominantly free of fibrillate interconnections. Particularly, the node segments being free of fibrillate interconnections have a length from 1 to 99 %, in particular from 2 to 80 %, preferably 5 to 70 %, more preferably 10 to 45 %, and especially preferred from 15 to 35 %, in relation to the total length of the nodes. Preferably, the node segments being free of fibrillate interconnections have a length from 1.5 to 795 µm, in particular from 3 to 600 µm, preferably 5 to 400 µm, more preferably 10 to 250 µm and especially preferred from 20 to 150 µm.

In a preferred embodiment, basically all nodes, in particular all nodes, at the surface of the article comprise segments that are free of fibrillate interconnections. Preferably, the node segments that are free of fibrillate interconnections are arranged in the form of a zone (area or sphere) at the surface of the porous article having a proportion from 2 to 30 %, in particular from 5 to 20 %, and preferred from 10 to 15 %, relating to the thickness, in particular wall thickness, of the porous article. This has the advantage, that body cells, in particular fibroblasts, cannot infiltrate the porous article in its entirety which might cause an undesired passage of the cells into a lumen of the porous article causing an undesired occlusion thereof. Preferably, the zone has a thickness from 4 to 300 µm, in particular from 20 to 150 µm, and preferred from 40 to 80 µm.

The node segments that are free of fibrillate interconnections may have fibrillate protrusions. Said protrusions may extend from the body of the nodes and node segments, respectively over a length from 1 to 150 µm, in particular from 10 to 100 µm, and preferred from 20 to 80 µm. Normally, the protrusions have a length less than the length of the interconnecting fibrils and/or internodal spaces of the microstructure. Accordingly, the protrusions normally offer a significantly reduced resistance to cellular ingrowth in comparison to fibrils that interconnect nodes.

As already mentioned, the microstructure is based on nodes that are interconnected by fibrils. Preferably, nodes being along their size, in particular length, preferably continuously interconnected by fibrils may be arranged in the form of a zone (area or sphere) having a proportion from 98 to 70%, in particular from 95 to 80 %, and preferred from 90 to 85 %, taken at the thickness, in particular wall thickness, of the porous article. The zone may have a thickness from 140 to 980 µm, in particular from 250 to 750 µm, and preferred from 425 to 475 µm. It is especially preferred that the zone of nodes being along their size, in particular length, preferably continuously interconnected by fibrils is beneath a zone of nodes having segments that are free of fibrillate interconnections. In a further preferred embodiment, the nodes being along their size, in particular length, continuously interconnected by fibrils are present at an inside surface of the porous article.

Preferably, the nodes of the microstructure have an elongate, in particular an ellipsoidal, shape. More preferably, the nodes have a relatively uniform shape and in particular a uniform size. The nodular size, particularly the nodular length, may range from 1 to 800 µm, in particular from 5 to 500 µm, and preferred from 10 to 300 µm. The interconnecting fibrils may have a bent, wavy or straight appearance. More preferably, fibrils that interconnect nodes have a straight and parallel appearance. Further, the fibrils may have a length from 1 to 150 µm, in particular from 20 to 80 µm. Furthermore, the fibrils may have a diameter that ranges from 0.1 to 10 µm, in particular from 0.5 to 5 µm. In a further embodiment, the nodes of the microstructure are essentially oriented in a direction perpendicular to the longitudinal and lateral direction of the porous article.

In a further embodiment, the microstructure includes nodes arranged in differing internodal spaces (spaces between the nodes). Preferably, the internodal spaces are arranged in a gradient. More preferably, internodal spaces increase, in particular gradually increase, from one surface to an opposing surface of the porous article. Typically, the nodes of the microstructure may have an internodal space from 1 to 150 µm, in particular from 10 to 100 µm, and preferred from 20 to 80 µm.

In a further embodiment, the porous article comprises a coating, preferably including an antimicrobial material, in particular selected from the group consisting of an antimicrobial metal, antimicrobial alloy, antimicrobial compound, antimicrobial salt and combinations thereof. Antimicrobial metals, antimicrobial alloys and/or antimicrobial metal compounds, in particular antimicrobial metal salts, are especially preferred. Typically, only the surface of the porous article, preferably only a part thereof, comprises the coating. More preferably, the node segments that are free of fibrillate interconnections are at least partially, in particular completely, coated, preferably with an antimicrobial material. In particular, only the node segments being free of fibrillate interconnections are at least partially, in particular completely, coated, preferably with an antimicrobial material. The remaining segments of these nodes are preferably interconnected by fibrils, i.e. comprise fibrillate interconnections. The remaining segments are in particular aligned (oriented) to a direction that is converse to an outside surface of the porous article.

Further, it is preferred that internodal spaces at the surface of the porous article are free of a coating, particularly free of a coating including an antimicrobial material. In other words, the coating preferably leaves the internodal spaces open at the surface of the porous article. The antimicrobial material may be selected from the group consisting of zirconium, copper, zinc, silver, gold, palladium, platinum, iridium, aluminium, nickel, tungsten, molybdenum, tantalum, titanium, iodine, alloys thereof, compounds, in particular salts, for instance oxides, thereof and combinations thereof. As used herein, the term "antimicrobial material" preferably refers to a material which inhibits the growth of micro-organisms, such as pathogenic bacteria, protists and/or fungi, which can cause infections within a patient.

Further, the coating, in particular including an antimicrobial material, may have a layer thickness from 100 to 4000 Å, in particular from 400 to 2500 Å, preferably from 800 to 1600 Å. Furthermore, the coating may have a proportion from 0.01 to 5.0 % by weight, in particular from 0.1 to 3.0 % by weight, and preferred from 0.3 to 1.5 % by weight, relating to the total weight of the porous article. Preferably, the coating is applied to the porous article in a depth from 1 to 150 µm, in particular from 20 to 100 µm, and preferred from 40 to 80 µm, taken at the surface, preferably taken at an outside surface, of the porous article.

In a further embodiment, the porous article comprises an adhesion-promoting agent. Typically, the adhesion-promoting agent is adjacent to the surface of the article. Preferably, the adhesion-promoting agent is arranged in the form of a layer. Further, the adhesion-promoting agent may be a metal, in particular titanium. The porous article may further comprise a species, in particular a layer thereof, which forms a barrier to degradation or diffusion of an adhesion-promoting agent and, for instance, impede galvanic interaction between an antimicrobial material and an adhesion-promoting agent. The species may be a metal, in particular palladium. Preferably, the barrier species is adjacent to the surface of the porous article and in particular interposed between an adhesion-promoting agent and an antimicrobial material.

In a further embodiment, the porous article may comprise a leak-proofing coating or impregnation. Such a coating and impregenation, respectively is typically designed on the surface of the article. Suitable materials for the coating and impregnation, respectively may be biopolymers and/or synthetic polymers, for example, copolymers. Suitable materials may be selected from the group consisting of collagen, gelatine, albumin, polyvinylalcohol, carboxymethylcellulose and combinations thereof. Further suitable materials may be selected from the group consisting of polylactide, polyglycolide, poly- ε-caprolactone, polytrimethylencarbonate, poly-para-dioxanone, copolymers thereof and combinations thereof. Typically, such a coating and impregnation, respectively may seal internodal spaces at the surface of the article. Due to the node segments that are free of fibrillate interconnections the afore-described coating and impregnation, respectively may stronger adhere to the surface of the article.

In a further preferred embodiment, the node segments that are free of fibrillate interconnections result from a surface treatment, in particular from a dry coating process, preferably from a physical vapour deposition process (PVD process), more preferably from an ion-beam-assisted deposition process (IBAD process), in particular of an antimicrobial material, preferably of an antimicrobial metal. More details thereof are described in the following description.

According to an especially preferred embodiment, the porous article is a tubular article, typically a hollow tubular article. In other words, the porous article is preferably designed as a tubing, typically having a lumen encased by the tubing wall. More preferably, the node segments that are free of fibrillate interconnections are present at the outside (exterior) surface of the tubular article. Further, it is preferred that nodes being along their size, in particular length, preferably continuously interconnected by fibrils are present at the inside (interior) surface of the tubular article. Pores, in particular internodal spaces, at the outside surface of the tubular article may differ from those at the inside surface of the tubular article. It is especially preferred, that the inside surface of the tubular article is essentially arranged smooth. In other words, the microstructure at the inside surface of the tubular article preferably offers no or only a negligible resistance to flow of blood, and consequently, platelet adhesion may be reduced. Furthermore, it is preferred that the inside surface may have a pore structure, in particular internodal spaces, that facilitates the passage of small molecular compounds, particularly of nutrients, biological agents and/or medical agents through the wall of the tubular article into the lumen thereof. Thus a nutrition supply to a neointima is possible that preferably lines the inside surface of the tubular article. Thus, it is possible to greatly reduce calcification of the neointima that may result from nutritional deficiency. Further, biological and/or medical agents may enter the lumen of the tubular article and, for instance, may help to prevent the manifestation of a thrombosis. Preferably, the microstructure at the outside surface of the tubular article comprises a pore structure, in particular internodal spaces, that promotes tissue ingrowth, in particular ingrowth of fibroblasts, from the outer periphery of the article. This contributes to a secure anchoring of the article with surrounding connective tissue. Furthermore, the aforementioned nutrient supply is essentially based on capillaries which densely develop on fully grown fibroblasts. Preferably, pore sizes, in particular internodal spaces, of the microstructure increase, particularly gradually increase, from the inside surface to the outside surface of the tubular article.

In a further embodiment, the porous article is designed as a tubular article having an internal diameter from 2 to 50 mm.

Further, the porous article may have a thickness, in particular wall thickness, from 0.1 to 1.0 mm, in particular from 0.25 to 0.75 mm, preferably from 0.4 to 0.6 mm.

In an alternative embodiment, the porous article is designed as a two-dimensionally shaped (planar) article. Preferably, the node segments being free of fibrillate interconnections are present only at one surface of the two-dimensional shaped article, in particular facilitating cellular ingrowth into the article from said one surface. The opposing surface preferably comprises a microstructure that is based on nodes being along their size, in particular length, continuously interconnected by fibrils, in particular preventing cellular ingrowth from said opposing surface and preferably avoiding post-surgical adhesion with said opposing surface. In a further embodiment, the porous article is designed as a mesh, in particular for the repair of hernias, or as a patch, in particular for hemostasis.

In a further preferred embodiment, the medical device is selected from the group consisting of tubular prostheses, catheters, stents, shunts, hernia meshes, prolaps meshes, and aconuresis slings. More preferably, the medical device according to the present invention is a tubular prosthesis, in particular a vascular prosthesis (vascular graft). More specifically, the medical device may be an arterial prosthesis or a vein prosthesis. However, it is especially preferred that the medical device is an arterial prosthesis.

Further, the medical device may be on hand in a sterile form and in particular tailored form.

The present invention further encompasses a method for the manufacture of a medical device according to the present invention comprising the step of subjecting a porous article of expanded polytetrafluoroethylene (ePTFE) having a microstructure of interspaced nodes interconnected by fibrils to a surface treatment forming nodes having segments that are free of fibrillate interconnections at a surface of the porous article, preferably at an outside surface thereof.

Typically, the porous article is provided as a tubular article, i.e. tubing, having an inside surface and an outside surface. It is especially preferred that only the outside surface of the tubular article is subjected to the surface treatment. Thus, the node segments being free of fibrillate interconnections are preferably formed at the outside surface of the tubular article. The microstructure at the inside surface of the tubular article is preferably not affected by the surface treatment.

In a preferred embodiment, the fibrils interconnecting the nodes are at least partially, in particular completely, open broken, disintegrated, in particular melted on, by the surface treatment. Insofar it is within the scope of the present invention that the microstructure at the surface of the article is at least partially, in particular completely, altered or modified by the surface treatment.

In a further preferred embodiment, the surface treatment additionally includes the introduction, in particular the deposition, of an antimicrobial material, in particular an antimicrobial metal, at the surface of the porous article. Thus, a porous article may be produced in one step having a microstructure at the surface thereof facilitating an easier tissue ingrowth and preferably having a long-lasting antimicrobial protection.

Preferably, the surface treatment is performed by means of a dry coating process, in particular by means of a physical vapour deposition process (PVD process). More preferably, the surface treatment is performed by means of an ion-beam-assisted deposition process (IBAD process). In general, ion-beam processes are low-temperature, high-technology processes with excellent quality control to achieve good adherence, ductility, reproducibility, reliability and thickness of deposition control at a high throughput and with no chemical residues, thus being both environmentally and occupationally a safe dependable technique. Typically, an ion-beam-assisted coating apparatus comprises a vacuum chamber system formed of a low-vacuum antechamber and a high vacuum processing chamber, air-tightly separated from each other by a gate movable between an opened position and a closed position. An ion source, which can be a bucket type ion source is mounted within the high-vacuum processing chamber, normally in a position diametrically opposed to the low-vacuum antechamber. The ion source is typically fed by one or more gases, such as argon, oxygen, neon, and/or helium, from a suitable gas supply source via a mess flow controller, regulating the rate of gas feed.

Further, an evaporator is also mounted in the high-vacuum processing chamber, normally in operative association with the ion source. The evaporator is designed to vaporize particular evaporants, more specifically metallic evaporents, so as to dry-coat a specific substrate, in particular a medical device, therewith, being assisted in the dry-coating by an ion-beam emanating from the ion source. Suitable evaporants include zirconium, copper, zinc, silver, gold, palladium, platinum, iridium, aluminium, nickel, tungsten, molybdenum, tantalum, titan and their respective alloys, oxides and compounds. Normally, a vapour shutter, designed to be rotated in and out of place of the evaporator, shields the substrates from the evaporants when in place. The substrate to be dry-coated is normally introduced into the vacuum chamber system with the aid of a suitable substrate holder. Preferably, the substrate holder is mounted for both rotational and translatory motion on a shaft and is introduced in the antechamber through a hinge-like mounted end-plate.

With respect to the above-made comments the IBAD process that is preferably scheduled by the present invention is typically performed in a suitable vacuum chamber system including a processing chamber. The required vacuum environment is normally created by means of a vacuum pump. Preferably, the ion-beam-assisted deposition is performed under a vacuum pressure of at least 10⁻⁴ torr, particularly of at least 10⁻⁵ torr. Further, the ion-beam-assisted deposition may be performed at a temperature < 150 °C. Moreover, the porous article may be exposed to an ion-beam energy from 5 to 10000 eV, in particular from 50 to 5000 eV, preferably 200 to 1000 eV. The above mentioned ion-beam energy is preferably intended to achieve an ion beam current density on the surface of the porous article from 0.1 to 500 µA/cm², in particular 1 to 250 µA/cm², preferably 10 to 80 µA/cm². In a further embodiment, the ion-beam-assisted deposition process is performed during a time period from 0.1 to 500 minutes, in particular from 1 to 200 minutes, preferably from 5 to 75 minutes. Further, the ion-beam-assisted deposition process may be performed applying a deposition rate from 0.1 to 50 Å(angstroms)/second, in particular from 1 to 25 Å(angstroms)/second, preferably from 2 to 10 Å(angstroms)/second.

In a particular preferred embodiment, the ion-beam-assisted deposition is carried out under the following process parameters: a vacuum pressure of at least 10⁻⁵ torr, an ion beam energy from 200 eV to 1000 eV, an ion beam current density from 10 to 80 µA/cm² and a deposition rate from 2 to 10 A(Angstroms)/second.

In a further embodiment, the surface treatment is performed applying a mechanical method, in particular brushing. In an alternative embodiment, the surface treatment is performed applying a thermal method, in particular employing laser techniques. Furthermore, sputtering or plasma treatment may be employed.

A further aspect of the present invention relates to a medical device, preferably a vascular prosthesis, obtained or obtainable according to one of the afore-described methods. For further details and advantages, reference is made to the previous description.

In the following, the present invention will be illustrated in more detail by a disclosure of preferred embodiments presented in figures, a description of the figures and a example. In the embodiments, the individual features may be realized exclusively or in combination with other features. Any described embodiment is given for the sole purpose of illustration and better understanding of the invention, and is no way to be interpreted as a limitation.

The figures depict the following:
Figure 1 is a schematic illustration of the node and fibril microstructure of conventional ePTFE;
Figure 2 is a schematic illustration of the node and fibril microstructure of ePTFE according to the present invention;
Figure 3: is a REM photo of the longitudinal cut of a ePTFE vascular prosthesis according to the present invention.

### Description of the figures

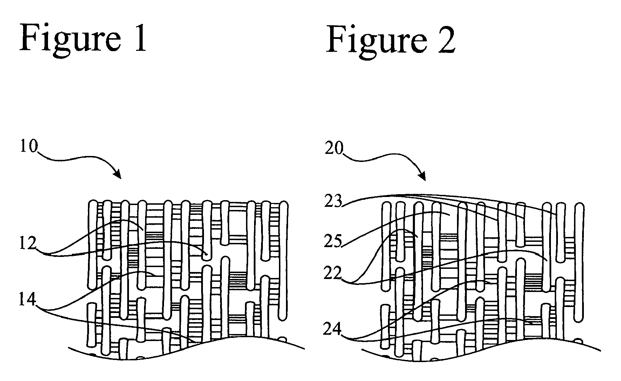

Figure 1 schematically illustrates the node fibril microstructure 10 of ePTFE that is known from the prior art and that is realized in conventional ePTFE prostheses. The microstructure 10 comprises interspaced nodes 12 that have typically an enlogate shape and are interconnected by fibrils 14. Importantly, also the nodes 12 being present at the surface 11 of the microstructure 10 are interconnected by fibrils. Dependent on the stretching and expanding conditions during the manufacture of ePTFE the form of the nodes 12 and in particular the appearance of the interconnecting fibrils 14 may vary. For example, the fibrils 14 may have a bent, wavy or - as illustrated in Figure 1 - a straight and in particular parallel - appearance.

Figure 2 schematically illustrates the microstructure 20 based on nodes 22 interconnected by fibrils 24 that is realized in a medical device according to the present invention. Accordingly, the microstructure 20 comprises at the surface 21, preferably at an outside surface, of the device nodes 22 having segments 23 that are free of fibrillate interconnections 24. In other words, internodal spaces 25 are present at the surface 21 of the device that are not restricted by fibrillate interconnections 24. Thus the medical device comprises enlarged openings or cavities, in particular pores, at its surface 21 allowing for an easier entry of connective tissue cells, in particular fibroblasts, into the device from its outer periphery. Thus, recovery of the original present anatomical situation in the body of a patient before implanting the device is accelerated. Furthermore an easier entry of connective tissue cells into the device contributes to a secure anchoring of the device in the body of a patient. Preferably, the node segments 23 are coated with an antimicrobial material.

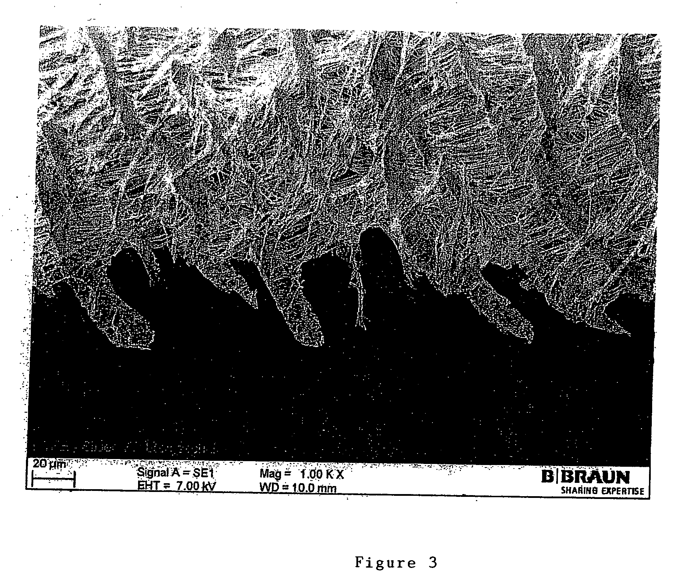

Figure 3 displays a REM picture of the longitudinal cut of an ePTFE vascular prosthesis according to the present invention. The REM picture clearly shows that the nodes at the outside surface of the vascular prosthesis comprise segments that are completely free of fibrillate interconnections forming enlarged cavities or openings at the outside surface of the prosthesis. The node segments preferably protrude from the outside surface and in particular confer the outside surface a more roughly structure, in particular a velour-like appearance. The specific microstructure at the outside surface of the prosthesis preferably depending on the aforementioned features facilitates the entry of body cells, in particular connective tissue cells, preferably fibroblasts, from the outer periphery of the vascular prosthesis. This leads to a secure anchoring of the prosthesis within the body of a patient and to a natural recovery of the implantation region.

### Example

Prostheses of ePTFE are clamped in a rotatable clamp device so that they hang freely as a bundle of parallel tubes with spaces between them. The clamp device is introduced into a vacuum chamber suitable for carrying out the IBAD technique, the ePTFE prostheses being vapour-deposited with silver and at the same time bombarded with argon ions. The coating operation is conducted until a silver layer thickness of 1300 Å is reached on the outside surface of the ePTFE prosthesis or the fibrils located there. If desired, a primary coating of other metals can be effected by vapour-deposition.

An ePTFE prosthesis with an internal diameter of 8 mm and a wall thickness of 500 µm coated in the above-described way has node segments at the outside surface which are free of fibrillate interconnections. The node segments being free of fibrillate interconnections form a zone having a layer thickness from 40 to 80 µm. The microstructure that is based on nodes interconnected by fibrils has a layer thickness from 420 to 460 µm. The silver coating typically have a film thickness of about 1300 Å resulting in a proportion of silver relative to the total weight in the range from 0.3 to 0.5 % by weight. The coating is applied to the ePTFE prosthesis in a depth from 40 to 80 µm.

## Claims

1. A medical device comprising a porous article of expanded polytetrafluoroethylene (ePTFE) having a microstructure comprising nodes interconnected by fibrils, **characterized in that** the microstructure at a surface of the article at least partially comprises nodes having segments that are free of fibrillate interconnections.

2. The medical device according to claim 1, **characterized in that** the node segments being free of fibrillate interconnections are aligned to the surface of the article, preferably form an outer periphery of the article.

3. The medical device according to claim 1 or 2, **characterized in that** the node segments that are free of fibrillate interconnections protrude, in particular like pillars, from the surface of the porous article.

4. The medical device according to one of the preceding claims, **characterized in that** the node segments being free of fibrillate interconnections have a length from 1 to 99 %, in particular from 2 to 80 %, preferably from 5 to 70 %, more preferably from 10 to 45 %, and especially preferred from 15 to 35 %, related to the total length of the nodes.

5. The medical device according to one of the preceding claims, **characterized in that** the node segments that are free of fibrillate interconnections are arranged in a zone having a proportion from 2 to 30 %, in particular from 5 to 20 %, and preferred from 10 to 15 %, taken at the thickness, in particular wall thickness, of the porous article.

6. The medical device according to one of the preceding claims, **characterized in that** the surface of the porous article comprises a coating including an antimicrobial material, in particular an antimicrobial metal, preferably silver.

7. The medical device according to one of the preceding claims, **characterized in that** the node segments being free of fibrillate interconnections are at least partially, in particular completely, coated with an antimicrobial material.

8. The medical device according to claim 6 or 7, **characterized in that** the coating including an antimicrobial material leaves internodal spaces open at the surface of the porous article.

9. The medical device according to one of the claims 6 to 8, **characterized in that** the coating has a layer thickness from 100 to 4000 Å, in particular from 400 to 2500 Å, and preferred from 800 to 1600 Å.

10. The medical device according to one of the claims 6 to 9, **characterized in that** the coating is applied to the porous article in a depth from 1 to 150 µm, in particular from 20 to 100 µm, and preferred from 40 to 80 µm, taken at the surface of the porous article.

11. The medical device according to one of the preceding claims, **characterized in that** the porous article is a tubular article wherein the node segments that are free of fibrillate interconnections are preferably present at the outside surface of the tubular article.

12. The medical device according to one of the preceding claims, **characterized in that** the medical device is a tubular prosthesis, in particular a vascular prosthesis, preferably an arterial prosthesis.

13. A method for the manufacture of a medical device, in particular according to one of the preceding claims, comprising the step of subjecting a porous article of expanded polytetraflouroethylene (ePTFE) having a microstructure of nodes interconnected by fibrils to a surface treatment forming nodes having segments that are free of fibrillate interconnections at a surface of the article.

14. The method according to claim 13, **characterized in that** the surface treatment is performed by means of a dry coating process, in particular by means of a physical vapour deposition process (PVD process), and preferred by means of an ion-beam-assisted deposition process (IBAD process).

15. A medical device obtained or obtainable by a method according to one of the claims 13 or 14.
